Europäisches Patentamt

European Patent Office

Office européen des brevets

(11) Publication number: **0 132 940**
A1

## (12) EUROPEAN PATENT APPLICATION

(21) Application number: **84304041.1**

(22) Date of filing: **15.06.84**

(51) Int. Cl.⁴: **A 61 B 5/14**
**A 61 M 5/00, A 61 M 5/32**

(30) Priority: **17.06.83 US 505438**

(43) Date of publication of application:
**13.02.85 Bulletin 85/7**

(84) Designated Contracting States:
**AT BE CH DE FR GB IT LI LU NL SE**

(71) Applicant: **Rangaswamy, Avvari**
**P.O. Box 426 13 Eastern Drive**
**Littleton North Carolina 27850(US)**

(72) Inventor: **Rangaswamy, Avvari**
**P.O. Box 426 13 Eastern Drive**
**Littleton North Carolina 27850(US)**

(74) Representative: **De Minvielle-Devaux, Ian Benedict**
**Peter et al,**
**CARPMAELS & RANSFORD 43, Bloomsbury Square**
**London WC1A 2RA(GB)**

(54) Arterial puncture unit.

(57) A blood vessel puncture unit (10) is disclosed wherein a pair of rectangular restraining bars (12) are arranged in a flat rectangular structure with a syringe holder (20) mounted on springs (18) above the restraining bars (12), when the flat structure is compressed against a patient's skin (32) over a blood vessel, with the restraining bars (12) perpendicular to the blood vessel, the restraining bars (12) fix the location of the blood vessel so that a syringe (22) mounted in the syringe holder (20) can be accurately guided to puncture the blood vessel. When blood is being drawn from a patient the syringe (22) mounted in the syringe holder (20) can include a rupturable reservoir (26) for release of an anticoagulant just prior to drawing blood.

FIG. 1.

## TITLE

### ARTERIAL PUNCTURE UNIT

### BACKGROUND OF THE INVENTION

This invention relates to a blood vessel stabilizer for use when a vein or artery puncture is being performed. In use the present invention fixes the location of the blood vessel to be punctured, and provides a guidable retainer for holding the syringe used for puncturing the restrained blood vessel. When blood is being drawn from a patient, the syringe used with the blood vessel stabilizer of the present invention includes a reservoir filled with an anticoagulant. This reservoir is ruptured just prior to drawing blood from the patient so as to permit the mixing of an appropriate amount of anticoagulant with the drawn blood.

Venipunctures and arterial punctures are among the more commonly performed medical procedures. Such surgical puncturing of a blood vessel to inject medications, or to withdraw fluids can be a difficult and painful procedure for many patients -- especially for children or frequently hospitalized patients.

The procedure for puncturing blood vessels with a syringe needle has remained substantially unchanged since the use of syringes was first instituted. Essentially, the procedure used for puncturing veins or arteries with syringe needles is to first visually locate the desired, or a convenient, blood vessel and then to manually force the syringed needle through the flesh surrounding the vessel until the vessel is punctured. Since the procedure is often performed when the patient is conscious, an adverse emotional effect is unavoidably produced because of the continual redirection of the syringe needle required for puncturing the vessel. No prior procedure or device to eliminate the requirement for continually correcting the location of the syringe needle to compensate for movement of the vessel until the vessel is punctured is known.

When blood is being drawn from a patient there is the added difficulty of assuring that a proper amount of anticoagulant material is mixed with the drawn blood. No prior device which incorporates a reservoir for anticoagulant material that is ruptured just prior to the drawing of blood by a syringe is known.

## SUMMARY OF THE INVENTION

The present invention provides a mechanism for restraining a blood vessel and aiding in controlled movement of a syringe for puncture of the blood vessel. This invention is especially useful for drawing blood from a patient and incorporates a reservoir system for mixing the proper amount of anticoagulant material with drawn blood. These features minimize the discomfort associated with such procedures, and is particularly useful in treating children and patients in whom it is hard to find a moderate size blood vessel.

To accomplish these purposes and others two restraining bars are assembled with two bridge braces into a flat structure having essentially right angular intersections. This flat structure is positioned over a selected blood vessel so that when the two restraining bars are pressed against the patient's skin the blood vessel is substantially restrained from further movement. Both proper positioning of the flat structure and a system for referencing the movement of the syringe needle are accomplished by use of two blood vessel overlay bars. These overlay bars are fixedly mounted at essentially the middle of the restraining bars and at right angles to the restraining bars, and when the flat structure is positioned over the selected blood vessel, prior to compression, these overlay bars are aligned with the longitudinal axis of the blood vessel. When the flat structure is compressed against the patient's skin, the space between the two overlay bars references the location of the restrained blood vessel.

Attached to the flat structure are two springs which support a syringe holder. By placing a syringe in the spring mounted syringe holder, the combination of the restraint of the blood vessel by the restraining bars, the blood vessel location reference provided by the overlay bars, and stabilization of the syringe provided by the spring tension results in an apparatus for efficiently performing a venipuncture or an arterial puncture with a minimum of traumatic effect on the patient. This apparatus when used for drawing blood is further fitted with a syringe incorporating a plastic bag reservoir containing anticoagulant material. The plastic bag is ruptured just prior to drawing blood by pressing the syringe plunger all the way into the hollow cylinder of the syringe. As so arranged in combination with the spring mounted syringe holder this syringe with its reservoir greatly facilitates the drawing of blood for analysis, such as bloodgas analysis.

## BRIEF DESCRIPTION OF THE DRAWINGS

In the drawings:

FIGURE 1 is a perspective view of the blood vessel unit according to the present invention; and

FIGURE 2 is a cross-sectional view of the blood vessel unit as illustrated in FIGURE 1.

## DETAILED DESCRIPTION OF THE INVENTION

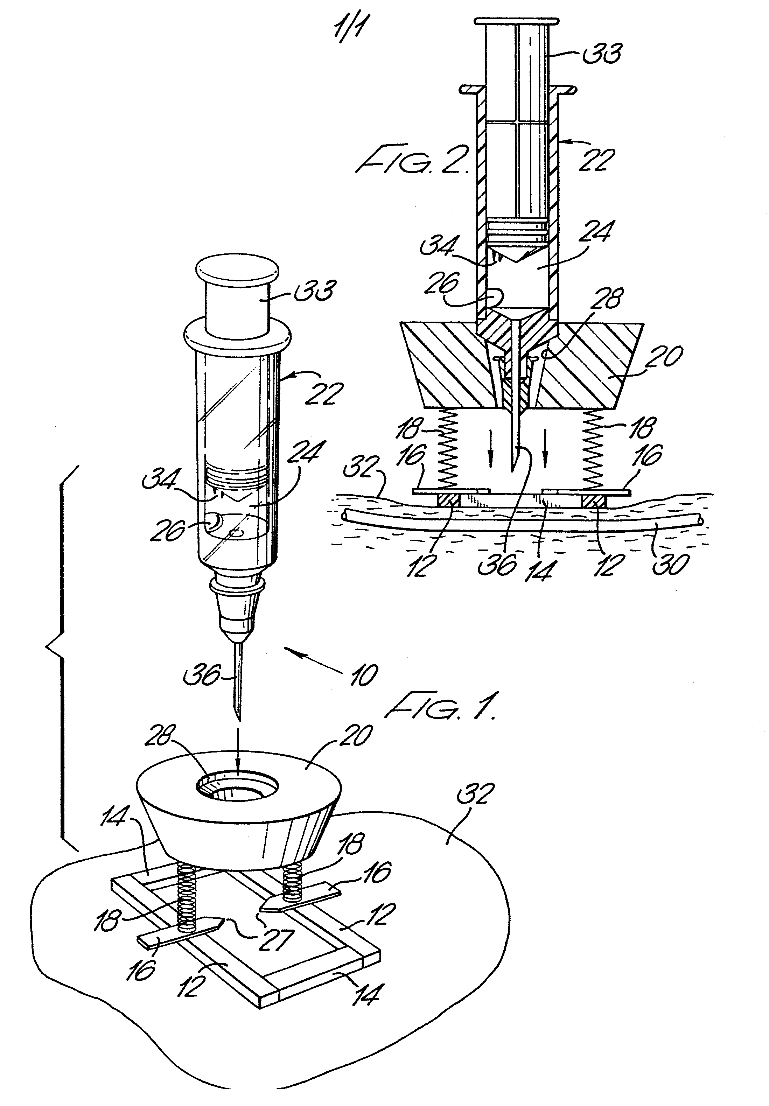

Referring now to the drawings, wherein corresponding components are designated by the same reference numerals throughout the various figures, a blood vessel puncture unit according to the invention is illustrated in FIG. 1. This unit is shown with a mounted syringe, and is generally designated by reference numeral 10.

Blood vessel puncture unit 10 includes restraining bars 12, bridge braces 14, blood vessel overlay bars 16, springs 18, syringe holder 20 and syringe 22. Within the hollow cylinder 24 of syringe 22 is a plastic bag reservoir 26. These structures, exclusive of the springs 18, can be fabricated from plastic by injection moulding or other expedient process known in the art. Such fabrication provides an inexpensive disposable unit.

The restraining bars 12 are fabricated in rectangular form as shown in FIG. 2, and are so arranged with bridge braces 14 to form a rigid rectangular flat structure. The blood vessel overlay bars 16 are fixedly attached to the restraining bars 12 at about the middle of the restraining bars 12 so that there is a gap of about one (1) to one and one half (1.5) centimeters between the ends 27 of blood vessel overlay bars 16. This gap between the ends 27 is located approximately half way between the restraining bars 12. Attached to the overlay bars 16 at the intersection of the restraining bars 12 and the overlay bars 16 are springs 18. At the end of springs 18, which are opposite restraining bars 12, is attached syringe holder 20. Syringe holder 20 is cylindrical in shape with a stepped aperture 28 as shown in FIG. 2. This stepped aperture 28 is configured so as to accept the distal end of syringe 20.

Use of blood vessel puncture unit 10 begins with the identification of a blood vessel 30 to be punctured. After blood vessel 30 is selected the blood vessel puncture unit 10 is oriented over the blood vessel with the overlay bars 16 essentially located over and parallel to longitudinal axis of blood vessel 30. This orientation assures that restraining bars 12 are so located as to have one of the restraining bars 12 perpendicular to the blood flow through the blood vessel 30 on the proximal (superior) side of the blood vessel puncture unit 10, and the other restraining bar 12 perpendicular to the blood flow through the blood vessel 30 on the distal (inferior) side of the blood vessel puncture unit 10. With this orientation preserved the blood vessel puncture unit 10 is compressed against the skin 32 of the patient so as to restrict motion of the blood vessel 30, and to fix the location of blood vessel 30 under the gap between ends 27 of the overlay bars 16.

- 6 -

Now with the position of syringe 22 controlled by syringe holder 20, the syringe plunger 33 can be fully inserted into the syringe hollow cylinder 24 so that the sharp points 34 rupture the plastic bag reservoir 26. This rupture of plastic bag reservoir 26 releases an anticoagulant material such as heparin. By use of a premeasured amount of anticoagulant material in plastic bag reservoir 26 the previously difficult problem of measuring the correct amount of anticoagulant material to be mixed with drawn blood is addressed. With syringe plunger 33 fully depressed into hollow cylinder 24 the syringe 22 mounted in syringe holder 20 can accurately be guided so that the syringe needle 36 punctures the patient's skin between ends 27 of overlay bars 16 and passes through the patient's flesh to puncture blood vessel 30. When blood vessel 30 is punctured syringe plunger 33 can be withdrawn from the distal end of syringe 22 and blood will enter syringe hollow cylinder 24 where it will mix with the anticoagulant material released from plastic bag reservoir 26.

The above discussion and related illustrations of the present invention are directed primarily to a preferred embodiment and practice of the invention. However, it is believed that numerous changes and modifications in the actual implementation of the concepts described herein will be apparent to those skilled in the art. For example, a syringe without a reservoir could be used, such changes and modifications may be made without departing from the scope of the invention as defined by the following claims.

CLAIMS

1. A blood vessel puncture unit, comprising:

(a) a pair of restraining bars fixedly interconnected with bridge braces to form an essentially flat structure;

(b) a syringe holder attached by spring means to said flat structure; and,

(c) a syringe attached to said syringe holder so that the syringe needle can be moved to the area between said restraining bars;

whereby said flat structure can be positioned over a blood vessel, and compression of said flat structure against the skin and flesh between said blood vessel and said flat structure essentially fixes the location of said blood vessel so that said syringe needle can be moved to puncture said blood vessel.

2. A blood vessel puncture unit as set forth in claim 1 in which a pair of blood vessel overlay bars are one each attached at approximately the mid-point of each restraining bar so that ends of said pair of blood vessel overlay bars are not touching but are fixedly spaced apart in the approximate center of said flat structure,

whereby said flat structure can be positioned over a blood vessel, with said pair of blood vessel overlay bars essentially oriented parallel to said blood vessel, and compression of said flat structure against the skin and flesh between said blood vessel and said flat structure essentially fixes the location of said blood vessel so that said syringe needle can be moved to puncture said blood vessel.

_- 2 ._

3.  A blood vessel puncture unit as set forth in claim 1 in which said spring means includes a pair of springs one each attached to said flat structure and to said syringe holder so that said pair of springs compliantly restrains movement of said syringe holder toward said flat structure.

4.  A blood vessel puncture unit as set forth in claim 1 in which a tapered aperture is positioned in said syringe holder, and said syringe can be located in said tapered aperture with said syringe needle generally directed toward the center of said flat structure, and said syringe in said syringe holder is compliantly restrained from movement toward said flat structure by said spring means.

5.  A blood vessel puncture unit as set forth in claim 1 in which said syringe includes at the distal end of the syringe in the syringe hollow cylinder a rupturable reservoir filled with a chemical agent, said rupturable reservoir is located so that when the syringe plunger is moved to the distal end of said syringe said rupturable reservoir is ruptured and said chemical agent is released for mixing with fluids drawn into said syringe hollow cylinder by movement of said syringe plunger from said distal end of said syringe.

FIG. 2.

FIG. 1.

**European Patent Office**

## EUROPEAN SEARCH REPORT

Application number

| | DOCUMENTS CONSIDERED TO BE RELEVANT | | EP 84304041.1 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A | US - A - 4 332 248 (T.N. DEVITIS) <br> * Fig. 6,7; column 3, lines 3-50 * <br> -- | 1 | A 61 B 5/14 <br> A 61 M 5/00 <br> A 61 M 5/32 |
| A | US - A - 2 238 323 (J.R. HOLLINGS-WORTH) <br> * Totality * <br> -- | 1,4 | |
| A | US - A - 3 612 051 (R.O. ARCE) <br> * Fig. 3,4; column 2, line 72 - column 3, line 27 * <br> -- | | |
| A | US - A - 1 943 120 (S. KABNICK) <br> * Totality * <br> ---- | 5 | |

| TECHNICAL FIELDS SEARCHED (Int. Cl. ³) |
|---|
| A 61 B  5/00 <br> A 61 B 17/00 <br> A 61 M  5/00 |

The present search report has been drawn up for all claims

| Place of search | Date of completion of the search | Examiner |
|---|---|---|
| VIENNA | 16-10-1984 | LUDWIG |